# EUROPÄISCHE PATENTSCHRIFT

(11) **EP 2 744 796 B1**
(45) Veröffentlichungstag und Bekanntmachung des Hinweises auf die Patenterteilung: **03.02.2016**
(21) Anmeldenummer: 12753924.5
(22) Anmeldetag: 16.08.2012
(51) Int. Cl.: C07D 257/04

(54) **ENERGETISCHE WIRKMASSE UMFASSEND EIN DIHYDROXYLAMMONIUMSALZ ODER DIAMMONIUMSALZ EINES BISTETRAZOLDIOLS**
ENERGETIC ACTIVE COMPOSITION COMPRISING A DIHYDROXYLAMMONIUM SALT OR DIAMMONIUM SALT OF A BISTETRAZOLEDIOL
MASSE ÉNERGÉTIQUE ACTIVE COMPRENANT UN SEL D'HYDROXYLE D'AMMONIUM OU SEL DE DIAMMONIUM D'UN BIS-TÉTRAZOLE-DIOL

(30) Priorität: 19.08.2011 DE 102011081254; 08.12.2011 DE 102011120745; 09.12.2011 US 201161568760 P
(43) Veröffentlichungstag der Anmeldung: 25.06.2014
(73) Patentinhaber: Ludwig-Maximilians-Universität München, 80539 München (DE)
(72) Erfinder: KLAPÖTKE, Thomas M., 81477 München (DE); FISCHER, Niko, 86154 Augsburg (DE); FISCHER, Dennis, 86424 Dinkelscherben (DE); PIERCEY, Davin G., Edmonton, Alberta T6W-1B6 (CA); STIERSTORFER, Jörg, 82237 Wörthsee (DE); REYMANN, Marius, 87746 Schlegelsberg (Erkheim) (DE)
(74) Vertreter: Ehnis, Tobias
(86) Internationale Anmeldenummer: PCT/EP2012/066023
(87) Internationale Veröffentlichungsnummer: WO 2013/026768

(56) Entgegenhaltungen:
- EP-A2- 2 377 840
- TSELINSKII, I.V. ET AL.: "Synthesis and Reactivity of Carbohydroximoyl Azides: I. Aliphatic and Aromatic Carbohydroximoyl Azides and 5-Substituted 1-Hydroxytetrazoles Based Thereon", RUSSIAN JOURNAL OF ORGANIC CHEMISTRY, Bd. 37, Nr. 3, 2001, Seiten 455-461, XP002684354, in der Anmeldung erwähnt

## Beschreibung

Die Erfindung betrifft eine energetische Wirkmasse umfassend ein Dihydroxylammoniumsalz oder Diammoniumsalz eines Bistetrazoldiols, eine Verwendung des Dihydroxylammoniumsalzes oder Diammoniumsalzes, ein Verfahren zur Herstellung des Dihydroxylammoniumsalzes oder Diammoniumsalzes und Bistetrazoldiole sowie Dihydroxylammoniumsalze und Diammoniumsalze davon.

Aus der US 6,040,453 A ist ein Verfahren zur Herstellung des Diammoniumsalzes von 5,5'-Bi-1H-Tetrazol unter Verwendung von Dicyan, Natriumazid, Ammoniumchlorid und Wasser als Reaktionsmedium bekannt. Dabei wird das Dicyan einer Natriumazid und Ammoniumchlorid enthaltenden wässrigen Lösung bei niedriger Temperatur zugesetzt. Anschließend wird die Mischung erhitzt. Das entstehende 5,5'-Bi-1H-Tetrazol-Diammoniumsalz fällt dabei in Form schwer löslicher Kristalle aus. Tselinskii, I. V. et al., Russian Journal of Organic Chemistry, Band 37, Nr. 3, 2001, Seiten 430 bis 436 offenbart die Synthese und Reaktivität von aliphatischen und aromatischen Carbohydroximoylaziden und darauf basierenden 5-substituierten 1-Hydroxytetrazolen.

Aus Göbel, M. et al., J. AM. CHEM. SOC. 2010, 132, Seiten 17216 bis 17226 ist die Oxidation von Nitrotetrazolat zum Nitrotetrazolat-2N-oxid-Anion und die Herstellung des Hydroxylammoniumsalzes davon bekannt. Das Hydroxylammoniumsalz zeigte in der theoretischen Berechnung bessere Detonationscharakteristika als der Sekundärsprengstoff HMX. Gemäß Seite Seite 17224, linke Spalte, zweiter Absatz der Druckschrift schließen jedoch die thermische Stabilität des Salzes und das extreme Zerfließen der freien Säure, die sich an Luft innerhalb weniger Minuten in absorbiertem Wasser löst, eine praktische Anwendung wahrscheinlich aus.

Weiterhin sind Nitramine als Sekundärsprengstoffe bekannt, wie beispielsweise Hexogen (RDX), Octogen (HMX) oder Hexanitroisowurtzitan (CL-20). Ein Nachteil dieser Nitramine und ihrer Reaktionsprodukte nach einer Detonation besteht in deren Toxizität und Umweltschädlichkeit. Weiterhin besteht ein Bedürfnis nach leistungsfähigeren Sekundärsprengstoffen. Diese sind zwar bereits bekannt, beispielsweise in Form von Dinitroazofuroxan oder Octanitrocuban. Ein Nachteil dieser Stoffe besteht in ihrer für Sekundärsprengstoffe hohen Sensitivität. Weiterhin ist deren Synthese sehr aufwändig und erfordert 10 oder mehr Reaktionsschritte.

Aufgabe der vorliegenden Erfindung ist es, eine alternative energetische Wirkmasse bereitzustellen, welche einfach herzustellen ist und eine hohe Leistungsfähigkeit bei sicherer Handhabbarkeit und vertretbarer Umweltschädlichkeit aufweist. Weiterhin sollen Bestandteile der Wirkmasse, eine Verwendung mindestens eines Bestandteils dieser Wirkmasse und ein Verfahren zur Herstellung eines solchen Bestandteils bereitgestellt und als Ausgangsstoffe oder Zwischenprodukte zur Erzeugung eines solchen Bestandteils geeignete Stoffe angegeben werden.

Die Aufgabe wird durch die Merkmale der Ansprüche 1, 2, 3 und 15 gelöst. Zweckmäßige Ausgestaltungen der Erfindung ergeben sich aus den Merkmalen der Ansprüche 4 bis 14. Erfindungsgemäß ist eine energetische Wirkmasse vorgesehen, die ein Dihydroxylammoniumsalz oder Diammoniumsalz von 5,5'-Bistetrazol-1,1'-diol, 5,5'-Bistetrazol-1,2'-diol oder 5,5'-Bistetrazol-2,2'-diol oder ein Gemisch aus mindestens zwei dieser Salze umfasst. Unter einer energetischen Wirkmasse wird hier eine nach deren Zündung deflagrativ oder detonativ reagierende Wirkmasse verstanden. Es kann sich dabei um eine pyrotechnische Wirkmasse handeln. Ein Vorteil der genannten Dihydroxylammoniumsalze und Diammoniumsalze ist, dass es sich dabei nicht um Nitramine handelt und somit auch bei deren Abbau in der Umwelt keine umweltschädlichen Nitrosamine entstehen. Die Umweltverträglichkeit dieser Salze ist deutlich besser als die Umweltverträglichkeit der genannten Nitramine und der Reaktionsprodukte der Nitramine.

Darüber hinaus hat es sich gezeigt, dass beispielsweise das Dihydroxylammoniumsalz Dihydroxylammonium-5,5'-bistetrazol-1,1'-diolat eine um 250 m/s höhere berechnete Detonationsgeschwindigkeit als CL-20 und eine um 700 m/s höhere berechnete Detonationsgeschwindigkeit als RDX aufweist. Das Diammoniumsalz Diammonium-5,5'-bistetrazol-1,1'-diolat weist eine ähnliche Detonationsgeschwindigkeit wie RDX auf. Beide Salze erfüllen damit die für einen Hochleistungssprengstoff geforderten Voraussetzungen. Die Berechnung der Detonationsgeschwindigkeiten erfolgte mit dem Programm EXPLO5, Version 5.05 (M. Sućeska, EXPLO5.04 program, Zagreb, Croatia, 2011; M. Suceska, Calculation of detonation parameters by EXPLO5 computer program, Materials Science Forum, 2004, 465-466, 325-330; M. Suceska, Calculation of the detonation properties of C-H-N-O explosives, Propellants, Explos., Pyrotech. 1991, 16, 197-202; M. Suceska, Evaluation of detonation energy from EXPLO5 computer code results, Propellants, Explos., Pyrotech. 1999, 24, 280-285; M. L. Hobbs, M. R. Baer, Proc. of the 10th Symp. (International) on Detonation, ONR 33395-12, Boston, MA, July 12-16, 1993, p. 409).

Weiterhin ist die Sensitivität des Dihydroxylammonium-5,5'-bistetrazol-1,1'-diolats und des Diammonium-5,5'-bistetrazol-1,1'-diolats jeweils geringer als diejenige von RDX. Die mit der Fallhammermethode bestimmte Schlagempfindlichkeit beträgt für RDX 7,5 J, so dass RDX zur Verwendung als Sekundärsprengstoff durch den Zusatz von Bindemitteln und Plastifizierungsmitteln desensitiviert werden muss, um handhabbar zu sein. Das Dihydroxylammoniumsalz von 5,5'-Bistetrazol-1,1'-diol (im Folgenden: "TKX50") weist dagegen bereits ohne Zusatzstoffe eine deutlich geringere Schlagempfindlichkeit von 20 J auf. Das Diammoniumsalz von 5,5'-Bistetrazol-1,1'-diol (im Folgenden: "ABTOX") weist ohne Zusatzstoffe sogar eine Schlagempfindlichkeit von 35 J auf. Dadurch sind TKX50 und ABTOX deutlich sicherer zu handhaben als RDX und erlauben eine einfachere Erfüllung der vorgeschriebenen Erfordernisse für insensitive Munition.

Darüber hinaus ist die Dichte von TKX50 höher als die Dichte von RDX und eine TKX50 enthaltende Wirkmasse kann wegen einem geringen Anteil von für eine Desensitivierung erforderlichen Zusatzstoffen eine deutlich höhere Dichte aufweisen als eine RDX enthaltende Wirkmasse. Dies bedeutet, dass in einem gegebenen Volumen eine höhere Masse einer TKX50 enthaltenden Wirkmasse untergebracht und damit eine höhere Leistung erreicht werden kann, als mit RDX.

Die Dichte von ABTOX entspricht in etwa der Dichte von RDX. Darüber hinaus weist ABTOX eine überragende thermische Stabilität auf. Es zersetzt sich bei einer Heizrate von 5 °C/min erst bei einer Temperatur von 290 °C und übersteigt damit bei weitem die Zersetzungstemperatur von RDX. ABTOX eignet sich daher zum Einsatz bei einer hohen Umgebungstemperatur, bei der RDX nicht eingesetzt werden kann.

Die Eigenschaften von TKX50 und ABTOX im Vergleich zu 2,4,6-Trinitrotoluol (2,4,6-TNT), RDX, β-HMX und ε-CL-20 sind in der folgenden Tabelle dargestellt:

| | 2, 4, 6-TNT | **RDX** | **β**-**HMX** | **ε-CL20** | **TKX50** | **ABTOX** |
|---|---|---|---|---|---|---|
| Formel | C₇H₅N₃O₆ | C₃H₆N₆O₆ | C₄H₈N₈O₈ | C₆H₆N₁₂O₁₂ | C₂H₈N₁₀O₄ | C₂H₈N₁₀O₂ |
| Molekulargewicht [g mol⁻¹] | 227,1 | 222,1 | 296,2 | 438,2 | 236,2 | 204,2 |
| IS [J]^{a} | 15 | 7,5 | 7 | 4 | 20 | 35 |
| FS [N]^{b} | 353 | 120 | 112 | 48 | 120 | 360 |
| ESD-Test [J]^{c} | - | 0,2 | 0,2 | - | 0,1 | 0,25 |
| *N* [%]^{d} | 18,5 | 37,8 | 37,8 | 38,3 | 59,3 | 68,6 |
| *Ω* [%]^{e} | -74,0 | -21,6 | -21,6 | -11,0 | -27,1 | -47,0 |
| T_{dec}. [°C]^{f} | 290 | 210 | 285 | 195 | 221 | 290 |
| Dichte [g cm⁻³]^{g} | 1,713 | 1,858 | 1, 944 | 2, 083 | 1, 918 | 1,800 |
| Δ_{f} *U*° / kJ kg^{-1 h} | -168 | 489 | 493 | 919 | 2006 | 1576 |
| -Δ*_{E}U*° [kJ kg⁻¹]ⁱ | 5258 | 6190 | 6185 | 6406 | 6025 | 4195 |
| *T_{E}* [K]^{j} | 3663 | 4232 | 4185 | 4616 | 3954 | 2931 |
| p_{C-J} [kbar]^{k} | 235 | 380 | 415 | 467 | 424 | 316 |
| D [m s⁻¹]^{l} | 7459 | 8983 | 9221 | 9445 | 9698 | 8809 |
| Gasvolumen [L kg⁻¹]^{m} | 569 | 734 | 729 | 666 | 846 | 843 |

| | | | | | | |
|---|---|---|---|---|---|---|
| ^{a} Schlagempfindlichkeit (gemessen mittels der Fallhammermethode gemäß der Bundesanstalt für Materialforschung und -prüfung, 1 von 6); ^{b} Reibempfindlichkeit (gemessen mit einem Reibapparat gemäß der Bundesanstalt für Materialforschung und -prüfung, 1 von 6); ^{c} gemessen mit der elektrostatischen Entladungsvorrichtung der Firma OZM Research s.r.o., Tschechien; ^{d} Stickstoffgehalt; ^{e} Sauerstoffbilanz; ^{f} Zersetzungstemperatur gemäß DSC (Differential Scanning Calorimetry)-Messung (5 °C pro Minute); ^{g} ermittelt mittels Röntgendiffraktometrie bei etwa 100 K; ^{h} Bildungsenergie berechnet mittels der CBS-4M-Methode; ⁱ Explosionsenergie; ^{j} Explosionstemperatur; ^{k} Detonationsdruck; ^{l} Detonationsgeschwindigkeit; ^{m} ermittelt unter der Annahme ausschließlich gasförmiger Reaktionsprodukte. | | | | | | |

Ein weiterer Vorteil der von der erfindungsgemäßen Wirkmasse umfassten Salze besteht darin, dass deren letzter Syntheseschritt in einer wässrigen Lösung erfolgen kann und dadurch verhältnismäßig sicher ist. Aus Wasser bzw. der wässrigen Lösung kristallisieren die Salze in idealen blockartigen Kristallen aus. Diese Kristalle sind für die Formulierung von Wirkmassen vorteilhaft, weil durch das geringe Oberfläche/Volumen-Verhältnis der Blöcke gegenüber den sich bei sonstigen Explosivstoffen üblicherweise bildenden Nadeln weniger Plastifizierungsmittel und Bindemittel benötigt werden, um einen sicher handhabbaren Wirkstoff bereitzustellen. Dadurch wird ein höherer Explosivstoffgehalt in der Wirkmasse und damit eine höhere Leistungsfähigkeit erreicht. Weiterhin ist die verhältnismäßig geringe Wasserlöslichkeit der Salze für deren Weiterverarbeitung vorteilhaft.

Bei einem Test der Leistungsfähigkeit mittels des sogenannten SSSRTs (Small Scale Shock Reactivity Test), bei dem untersucht wird, wie weit ein Aluminium-Block durch einen zu untersuchenden Sprengstoff bei dessen Detonation ausgebeult wird, zeigte es sich, dass TKX50 nach dessen Zündung eine ähnliche Leistungsfähigkeit wie β-HMX und eine höhere Leistungsfähigkeit als RDX aufweist. Bedingt durch eine nur teilweise erfolgte Detonation zeigte ABTOX beim SSSRT eine geringere Leistungsfähigkeit als RDX. Die nur teilweise erfolgte Detonation zeigt, dass ABTOX sehr sicher zu handhaben ist, weil für dessen vollständige Zündung ein sogenannter Boostersprengstoff erforderlich ist.

Da bekannt ist, dass beim SSSRT weniger sensitive Explosivstoffe eine größere Menge benötigen, um ihre Leistungsfähigkeit zu zeigen und sensitivere Explosivstoffe mit geringerer Leistungsfähigkeit eine scheinbar höhere Leistungsfähigkeit zeigen, kann davon ausgegangen werden, dass die tatsächlichen Leistungsfähigkeiten von TKX50 und ABTOX die Leistungsfähigkeit von β-HMX übersteigen.

Das Dihydroxylammoniumsalz von 5,5'-Bistetrazol-2,2'-diol weist gegenüber TKX50 eine etwas geringere thermische Stabilität, jedoch ebenso wie TKX50 eine verhältnismäßig hohe Dichte auf. Die hohe Dichte ist für leistungsfähige Sekundärsprengstoffe ein entscheidendes Kriterium. Das Dihydroxylammoniumsalz von 5,5'-Bistetrazol-2,2'-diol weist eine höhere Sensitivität als TKX50 auf und ist damit nicht nur als Sekundärsprengstoff, sondern auch als Boostersprengstoff geeignet. Ein Boostersprengstoff ist ein Sprengstoff, der zur Verstärkung der Wirkung eines anderen Sprengstoffs dient und dessen Sensitivität und Initiierbarkeit im Vergleich zu einem Primärsprengstoff niedriger und im Vergleich zu einem Sekundärsprengstoff höher ist.

Die vorteilhaften Eigenschaften der genannten Dihydroxylammoniumsalze und Diammoniumsalze waren so nicht zu erwarten. Gemäß Göbel, M. et al., J. AM. CHEM. SOC. 2010, 131, Seite 17224, linke Spalte, zweiter Absatz schließen die thermische Stabilität und das extreme Zerfließen der freien Säure eine praktische Anwendung wahrscheinlich aus. Bei dem gemäß dieser Veröffentlichung untersuchten Hydroxylammoniumsalz des Nitrotetrazolat-2N-oxids betrug die Zersetzungstemperatur lediglich 157 °C. Da sich wasserfreies 5,5'-Bistetrazol-1,1'-diol bei Tests als verhältnismäßig instabil und auf Grund großer Empfindlichkeit gegenüber Schlag, Reibung und elektrostatischer Entladung als Sprengstoff kaum als sicher handhabbar erwiesen hat, konnte der Fachmann nicht davon ausgehen, dass die von der erfindungsgemäßen Wirkmasse umfassten Dihydroxylammoniumsalze und Diammoniumsalze derart überragende Eigenschaften als Sprengstoff aufweisen würden.

Die Erfindung betrifft weiterhin die Verwendung eines Dihydroxylammoniumsalzes oder Diammoniumsalzes von 5,5'-Bistetrazol-1,1'-diol, 5,5'-Bistetrazol-1,2'-diol oder 5,5'-Bistetrazol-2,2'-diol oder eines Gemischs aus mindestens zwei dieser Salze als Sprengstoff, insbesondere als Sekundärsprengstoff. Die Sensitivität des Dihydroxylammoniumsalzes von 5,5'-Bistetrazol-2,2'-diol hat sich als hoch genug erwiesen, um sogar als Boostersprengstoff eingesetzt werden zu können.

Weiterhin betrifft die Erfindung ein Verfahren zur Herstellung eines Dihydroxylammoniumsalzes oder Diammoniumsalzes von 5,5'-Bistetrazol-1,1'-diol, 5,5'-Bistetrazol-1,2'-diol oder 5,5'-Bistetrazol-2,2'-diol oder eines Gemischs aus mindestens zwei dieser Salze mit folgenden Schritten:
a) Oxidieren von 5,5'-Bistetrazol zu einem Gemisch aus 5,5'-Bistetrazol-1,1'-diol, 5,5'-Bistetrazol-1,2'-diol und 5,5'-Bistetrazol-2,2'-diol oder
   Förderung der Isomerisierung von Diazidoglyoxim zu 5,5'-Bistetrazol-1,1'-diol oder einem 5,5'-Bistetrazol-1,1'-diolat,
b) Inkubation des gemäß Schritt a) erhaltenen Reaktionsprodukts mit Hydroxylamin, Hydroxylammoniumionen, Ammoniumionen oder Ammoniak in wässriger Lösung und
c) Absondern des dabei erhaltenen Präzipitats.

Bei dem gemäß Schritt a) erhaltenen Reaktionsprodukt kann es sich um das Gemisch gemäß Schritt a) oder das 5,5'-Bistetrazol-1,1'-diol handeln. Das Oxidieren gemäß Schritt a) kann durch Zusatz von 2KHSO₅·KHSO₄·K₂SO₄ oder einer anderen anorganischen oder organischen Peroxosäure oder hypofluoriger Säure oder einem anderen Sauerstoffüberträger zum 5,5'-Bistetrazol erfolgen. 2KHSO_{5·}KHSO₄·K₂SO₄ wird unter dem Handelsnamen "Oxone" von der Firma DuPont vertrieben. Der Zusatz von Oxone oder der anderen anorganischen oder organischen Peroxosäure oder hypofluoriger Säure oder dem anderen Sauerstoffüberträger erfolgt vorzugsweise im Überschuss, um eine vollständige Oxidation des 5,5'-Bistetrazols sicherzustellen. Beim Einsatz von Oxone im Überschuss entsteht das genannte Gemisch mit einem deutlichen Überschuss an 5,5'-Bistetrazol-2,2'-diol. Vorzugsweise erfolgt das Oxidieren in einer auf einen pH-Wert zwischen 5 und 8, insbesondere zwischen 7 und 7,5, gepufferten wässrigen Lösung. Das Puffern kann z. B. mittels Trinatriumphosphat erfolgen. Bei dem genannten pH-Wert liegt das 5,5'-Bistetrazol überwiegend deprotoniert vor. Dadurch wird ein für eine Oxidation durch Oxone erforderlicher nukleophiler Angriff des 5,5'-Bistetrazols am Peroxomonosulfat begünstigt.

Zur Erhöhung der Reinheit des Reaktionsprodukts kann das bei Schritt a) durch Oxidieren von 5,5'-Bistetrazol erhaltene Reaktionsprodukt angesäuert und anschließend mit einem organischen Extraktionsmittel, insbesondere Ether, extrahiert werden. Bei dem hier und im Folgenden genannten Ether handelt es sich insbesondere um Diethylether. Danach kann das Reaktionsprodukt, insbesondere durch Evaporation, aus dem Extrakt gewonnen werden. Zur weiteren Erhöhung der Reinheit kann der nach Evaporation verbleibende Rückstand rekristallisiert werden. Als dazu besonders geeignetes Lösungsmittel hat sich Methanol erwiesen.

Das Dihydroxylammoniumsalz und das Diammoniumsalz von 5,5'-Bistetrazol-1,1'-diol haben sich in Wasser als deutlich weniger löslich erwiesen als die Dihydroxylammoniumsalze und Diammoniumsalze von 5,5'-Bistetrazol-1,2'-diol und 5,5'-Bistetrazol-2,2'-diol. Um aus dem durch Oxidieren entstehenden Gemisch aus 5,5'-Bistetrazol-1,1'-diol, 5,5'-Bistetrazol-1,2'-diol und 5,5'-Bistetrazol-2,2'-diol spezifisch das Dihydroxylammoniumsalz oder Diammoniumsalz von 5,5'-Bistetrazol-1,1'-diol zu präzipitieren, genügt auf Grund der unterschiedlichen Löslichkeiten der aus dem Gemisch präzipitierbaren Dihydroxylammoniumsalze und Diammoniumsalze das Zusetzen des Hydroxylamins bzw. Ammoniaks zu der wässrigen Lösung.

Um die Dihydroxylammoniumsalze und Diammoniumsalze von 5,5'-Bistetrazol-1,2'-diol und 5,5'-Bistetrazol-2,2'-diol zu präzipitieren kann die wässrige Lösung nach der Präzipitation des Dihydroxylammoniumsalzes oder Diammoniumsalzes von 5,5'-Bistetrazol-1,1'-diol, insbesondere unter Zusatz von weiterem Hydroxylamin oder Ammoniak oder den Hydroxylammonium- oder Ammoniumionen, durch Evaporation eingeengt werden. Dadurch kann zumindest das Löslichkeitsprodukte des Dihydroxylammoniumsalzes oder Diammoniumsalzes von 5,5'-Bistetrazol-1,2'-diol oder 5,5'-Bistetrazol-2,2'-diol überschritten werden, so dass das Dihydroxylammoniumsalz oder Diammoniumsalz präzipitiert. Alternativ kann das Dihydroxylammoniumsalz oder Diammoniumsalz aus dem aus der Evaporation resultierenden Rückstand durch Umkristallisation, insbesondere aus einem Ethanol-Wasser-Gemisch, gewonnen werden.

Da durch Oxidieren von 5,5'-Bistetrazol das 5,5'-Bistetrazol-1,1'-diol nur in geringer Menge im Verhältnis zum 5,5'-Bistetrazol-2,2'-diol entsteht, hat es sich als vorteilhaft erwiesen, die Isomerisierung von Diazidoglyoxim zu Bistetrazol-1,1'-diol zu fördern, weil dabei kein 5,5'-Bistetrazol-1,2'-diol und kein 5,5'-Bistetrazol-2,2'-diol entsteht. Die Förderung der Isomerisierung kann durch Acylierung, insbesondere mittels Acetylchlorid, oder durch Inkubation mit gasförmigem HCl, insbesondere in Ether als Lösungsmittel oder in einem Ether enthaltenden Lösungsmittel, erfolgen. Das Diazidoglyoxim kann durch Reaktion von Dichloroglyoxim mit einem Azid erzeugt werden, wobei ein Chlor-Azid-Austausch erfolgt. Bei dem Azid kann es sich beispielsweise um Natriumazid handeln. Als Lösungsmittel kann dabei Dimethylformamid (DMF) verwendet werden. Das Dichloroglyoxim kann durch Reaktion von Glyoxim mit Chlor, beispielsweise in Ethanol als Lösungsmittel, erzeugt werden. Das Glyoxim wiederum kann durch Reaktion von Glyoxal mit Hydroxylamin erzeugt werden.

Bei einer vorteilhaften Ausgestaltung des Verfahrens erfolgt die Reaktion von dem Dichloroglyoxim mit dem Azid in einem nichtwässrigen Lösungsmittel, insbesondere Dimethylformamid (DMF) oder N-Methyl-2-pyrrolidon (NMP). Anschließend wird das Lösungsmittel mit dem dabei entstandenen Diazidoglyoxim mit dem Ether gemischt und mit dem gasförmigen HCl inkubiert. Der Vorteil dieses Verfahrens besteht darin, dass das Diazidoglyoxim nicht als Zwischenstufe isoliert werden muss, sondern in Lösung verbleibt. Da Diazidoglyoxim explosiv ist, wird das Herstellungsverfahren dadurch wesentlich sicherer und durch den Wegfall eines Isolationsschritts auch einfacher und kostengünstiger durchzuführen.

Es ist weiterhin möglich, vor Schritt c) den Ether und das HCl abzudampfen und, sofern vorhanden, das DMF abzudampfen. Das Abdampfen von Ether und HCl kann ggf. durch Zusatz von dann ebenfalls abzudampfendem H₂O erleichtert werden. Im Falle von DMF als Lösungsmittel wird eine Mischung aus Dimethylammonium-5,5'-bistetrazol-1,1'-diolat und einem 5,5'-Bistetrazol-1,1'-diolat, das mindestens ein Gegenion des dem Chlor-Azid-Austausch dienenden Azids umfasst, und im Falle von NMP als Lösungsmittel ein 5,5'-Bistetrazol-1,1'-diol enthaltender Rückstand erhalten.

Die genannte Mischung kann in H₂O gelöst werden, wobei anschließend ein Hydroxylammoniumsalz, insbesondere Hydroxylammoniumchlorid, zugesetzt wird, so dass das Dihydroxylammoniumsalz von 5,5'-Bistetrazol-1,1'-diol als das Präzipitat erhalten wird.

Der 5,5'-Bistetrazol-1,1'-diol enthaltende Rückstand kann in einer wässrigen Alkalihydroxid-Lösung aufgenommen und das ausfallende Alkali-5,5'-bistetrazol-1,1'-diolat abgesondert und in H₂O gelöst werden. Anschließend kann ein Hydroxylammoniumsalz, insbesondere Hydroxylammoniumchlorid, zugesetzt werden, so dass das Dihydroxylammoniumsalz von 5,5'-Bistetrazol-1,1'-diol als das Präzipitat erhalten wird.

Alternativ kann bei den obigen Verfahren statt des Hydroxylammoniumsalzes auch ein Ammoniumsalz, insbesondere Ammoniumchlorid, zugesetzt werden, so dass das Diammoniumsalz von 5,5'-Bistetrazol-1,1'-diol als das Präzipitat erhalten wird.

Die Erfindung betrifft weiterhin 5,5'-Bistetrazol-1,2'-diol und 5,5'-Bistetrazol-2,2'-diol. Beide Stoffe sind jeweils als Ausgangsstoff oder Zwischenprodukt zur Erzeugung des jeweiligen Dihydroxylammoniumsalzes oder Diammoniumsalzes davon geeignet. Darüber hinaus betrifft die Erfindung die Dihydroxylammoniumsalze und Diammoniumsalze von 5,5'-Bistetrazol-1,2'-diol und 5,5'-Bistetrazol-2,2'-diol.

Nachfolgend wird die Erfindung anhand von Ausführungsbeispielen näher erläutert. Es zeigen:
- Fig. 1: ein Reaktionsschema eines ersten Syntheseverfahrens zur Erzeugung des Dihydroxylammoniumsalzes von 5,5'-Bistetrazol-1,1'-diol,
- Fig. 2: ein Reaktionsschema eines zweiten Syntheseverfahrens zur Erzeugung des Dihydroxylammoniumsalzes von 5,5'-Bistetrazol-1,1'-diol und
- Fig. 3: ein Reaktionsschema eines Syntheseverfahrens zur Erzeugung des Diammoniumsalzes von 5,5'-Bistetrazol-1,1'-diol.

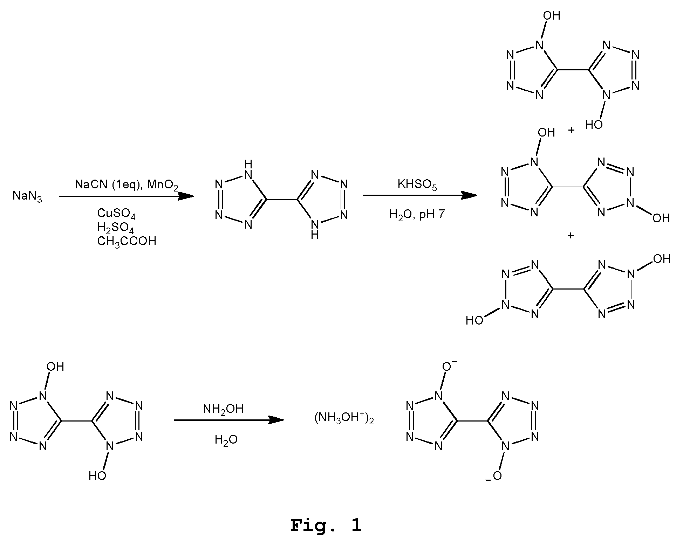

Zur Durchführung des ersten Verfahrens zur Synthese von Dihydroxylammonium-5,5'-bistetrazol-1,1'-diolat gemäß Fig. 1 kann wie folgt vorgegangen werden:
3,0 g 5,5'-Bistetrazol (21,7 mmol) werden in 200 ml Wasser gelöst. 80,0 g Oxone (109 mmol, 5 eq) werden zu der entstandenen klaren Lösung hinzugefügt und die resultierende Lösung wird mit Trinatriumphosphat auf einen pH-Wert von 7 gepuffert. Das Gemisch wird für 5 Stunden bei Raumtemperatur gerührt und dann mit konzentrierter Schwefelsäure angesäuert. Das Reaktionsprodukt wird mittels Ether extrahiert. Durch Evaporation des Ethers wird das Rohprodukt als leicht gelber Feststoff erhalten. Der Feststoff wird in Methanol gelöst und daraus rekristallisiert, um zurückgebliebene Sulfate oder Phosphate daraus zu entfernen. Die Reaktion ergibt eine Mischung aus 5,5'-Bistetrazol-1,1'-diol, 5,5'-Bistetrazol-2,2'-diol und 5,5'-Bistetrazol-1,2'-diol in einer Gesamtausbeute von 71% (2,60 g, 15,3 mmol). Dabei ist das 2,2'-Isomer das Hauptprodukt.

1,7 g des Isomerengemischs (10 mmol) werden in 20 ml heißem Wasser gelöst. Eine wässrige Lösung von 50% (w/w) Hydroxylamin (1,23 g, 20 mmol) werden zu der Lösung zugesetzt. Dabei bildet sich unmittelbar ein farbloses Präzipitat. Das Präzipitat wird durch Erwärmen der Mischung wieder gelöst. Beim langsamen Abkühlen präzipitiert das Dihydroxylammoniumsalz von 5,5'-Bistetrazol-1,1'-diol. Das Salz wird durch Filtrieren abgesondert. Das Salz wird dann in Wasser gelöst und daraus rekristallisiert, um verbliebenes 1,2'-Isomer und 2,2'-Isomer, die beide eine bessere Löslichkeit in Wasser aufweisen als das 1,1'-Isomer, zu entfernen.

Das Diammoniumsalz von 5,5'-Bistetrazol-1,1'-diol kann in analoger Weise aus dem in Wasser gelösten Isomerengemisch gewonnen werden. Dazu wird gasförmiges Ammoniak in die wässrige Lösung eingeleitet oder der wässrigen Lösung eine wässrige Ammoniaklösung zugesetzt. Dabei bildet sich unmittelbar ein Präzipitat, das durch Erwärmen der Mischung wieder gelöst wird. Beim langsamen Abkühlen präzipitiert das Diammoniumsalz von 5,5'-Bistetrazol-1,1'-diol. Das Salz wird durch Filtrieren abgesondert und dann in Wasser gelöst und daraus rekristallisiert, um verbliebenes 1,2'-Isomer und 2,2'-Isomer, die beide eine bessere Löslichkeit in Wasser aufweisen als das 1,1'-Isomer, zu entfernen.

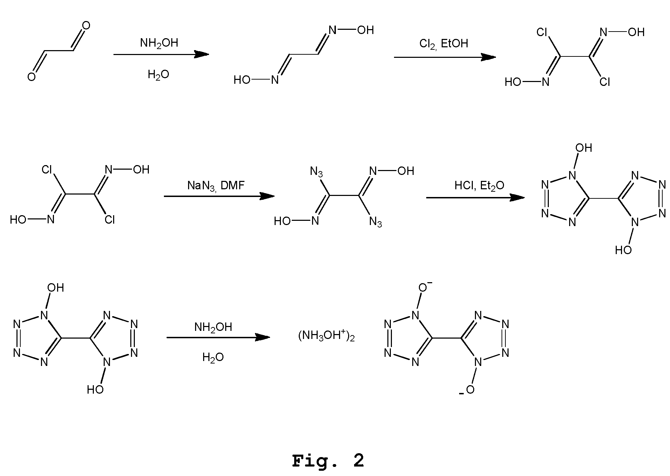

Bedingt durch die vorwiegende Bildung des 2,2'-Isomers bei der Oxidation des 5,5'-Bistetrazols kann das Dihydroxylammoniumsalz von 5,5'-Bistetrazol-1,1'-diol beim obigen Verfahren nur in verhältnismäßig geringer Ausbeute von 13% (0,31 g, 1,3 mmol) erhalten werden. Um eine höhere Ausbeute zu erhalten, kann die Synthese gemäß dem in Fig. 2 dargestellten Reaktionsschema durchgeführt werden. Dazu wird zunächst Dichloroglyoxim synthetisiert und daraus, wie in Tselinskii, I. V. et al., Russian Journal of Organic Chemistry, Band 37, Nr. 3, 2001, Seiten 430 bis 436 beschrieben, 5,5'-Bistetrazol-1,1'-diol als Dihydrat hergestellt. 2,06 g (10 mmol) des so erhaltenen Dihydrats werden in 50 ml warmem Wasser gelöst. Dazu wird eine 50%ige (w/w) Hydroxylaminlösung (1,32 g, 20 mmol) zugesetzt. Beim Abkühlen der Lösung auf Raumtemperatur fällt das Dihydroxylammoniumsalz kristallförmig aus. Es wird durch Filtration abgesondert und luftgetrocknet. Die Ausbeute beträgt 82%.

Zur Herstellung des Dihydroxylammoniumsalz bzw. Diammoniumsalzes von 5,5'-Bistetrazol-2,2'-diol wird wie bei der Synthese gemäß Fig. 1 vorgegangen. Nachdem beim Abkühlen des Gemischs das Dihydroxylammoniumsalz bzw. Diammoniumsalz des 1,1'-Isomers präzipitiert ist, kann das Dihydroxylammoniumsalz bzw. Diammoniumsalz von 5,5'-Bistetrazol-2,2'-diol durch Evaporation des Lösungsmittels und Umkristallisation des hieraus erhaltenen Rückstandes aus einem Ethanol-Wasser-Gemisch gewonnen werden.

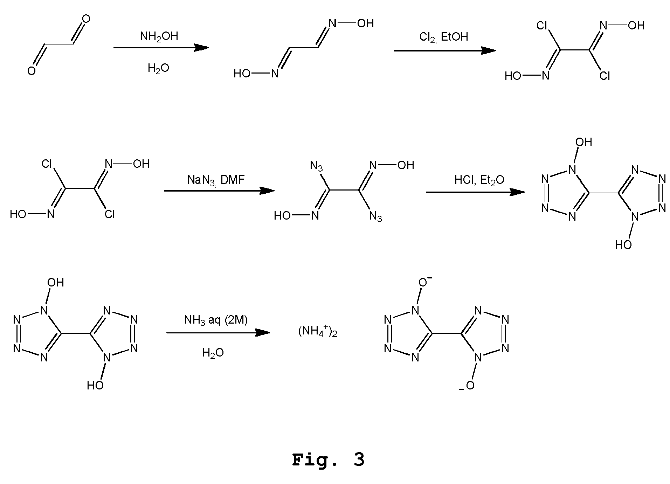

Die Synthese des Diammoniumsalzes von 5,5'-Bistetrazol-1,1'-diol kann auch gemäß dem in Fig. 3 dargestellten Reaktionsschema durchgeführt werden. Dazu wird zunächst Dichloroglyoxim synthetisiert und daraus, wie in Tselinskii, I. V. et al., Russian Journal of Organic Chemistry, Band 37, Nr. 3, 2001, Seiten 430 bis 436 beschrieben, 5,5'-Bistetrazol-1,1'-diol als Dihydrat hergestellt. 2,06 g (10 mmol) des so erhaltenen Dihydrats werden in 10 ml einer 2 M wässrigen Ammoniaklösung suspendiert. Nach Zusatz von 90 ml Wasser wird die Mischung auf ihren Siedepunkt erwärmt. Dabei entsteht eine klare Lösung. Beim Abkühlen der Lösung auf Raumtemperatur fällt das Diammoniumsalz kristallförmig aus. Es wird durch Filtration abgesondert und luftgetrocknet. Die Ausbeute beträgt 1,14 g (5,57 mmol, 56%).

### Weitere Syntheseverfahren:

TKX50: Dichlorglyoxim (785 mg, 5 mmol) wird bei Raumtemperatur in 10 ml N,N'-Dimethylformamid (DMF) gelöst. Die Lösung wird auf 0°C gekühlt und NaN₃ (715 mg, 11 mmol) zugegeben. Die Mischung wird bei 0°C 40 min gerührt, wobei NaCl ausfällt und Diazidoglyoxim in Lösung bleibt. Die Mischung wird in einen Kolben, in den 100 ml Diethylether bei 0°C vorgelegt wurden, überführt und HCl-Gas eingeleitet, wobei der Kolben ständig im Salz-Eis-Bad gekühlt wird und die Temperatur 20°C nicht überschreiten soll. Wenn die Temperatur trotz weiterer Gaseinleitung auf 0-5°C zurückgeht, ist HCl-Sättigung der Etherphase erreicht. Ein Niederschlag, welcher sich bei HCl-Einleitung bildet, agglomeriert zuerst und wird bei zunehmender HCl-Sättigung resuspendiert. Der Kolben wird mit einem Stopfen dicht verschlossen und die Mischung wird über Nacht bei Raumtemperatur unter leichtem HCl-Überdruck, welcher sich im Kolben aufgrund der Erwärmung auf Raumtemperatur bildet, weitergerührt. Der Druck wird abgelassen und die Mischung in ein offenes Gefäß überführt, so dass Diethylether und HCl entweder über Nacht bei Raumtemperatur oder in 1-2 h bei 50°C abdampfen kann. Nachdem der größte Teil des Ethers abgedampft ist, werden 50 ml Wasser zugegeben, wobei eine klare Lösung entsteht. Das Wasser wird am Rotationsverdampfer entfernt und das übrige DMF wird im Hochvakuum abgezogen, wobei man eine Mischung aus Dimethylammonium-5,5'-bistetrazol-1,1'-diolat und Dinatrium-5,5'-bistetrazol-1,1'-diolattetrahydrat als farblosen Feststoff erhält. Der Feststoff wird im kleinstmöglichen Volumen kochenden Wassers gelöst (ca. 10 ml) und Hydroxylammoniumchlorid (750 mg, 10,8 mmol, 2,16 eq) als konzentrierte wässrige Lösung zugegeben. TKX50 fällt aus der Lösung in 74,6% (882 mg, 3,73 mmol) Ausbeute aus. Es kann abgenutscht, mit wenig kaltem Wasser gewaschen und an der Luft getrocknet werden.

ABTOX: Die Synthesevorschrift orientiert sich an der Synthesevorschrift von TKX50, bis die Mischung aus Dimethylammonium-5,5'-bistetrazol-1,1'-diolat und Dinatrium-5,5'-bistetrazol-1,1'-diolattetrahydrat erhalten wird. Es wird wiederum im kleinstmöglichen Volumen kochenden Wassers gelöst (ca. 10 ml) und Ammoniumchlorid (800 mg, 15,0 mmol, 3eq.) als konzentrierte wässrige Lösung zugegeben. Im Falle von ABTOX muss ein etwas größerer Überschuss Ammoniumsalz zugegeben werden, da die Wasserlöslichkeit von ABTOX etwas größer als die von TKX50 ist. Ebenso muss das Volumen der ABTOX und Dimethylammoniumchlorid enthaltenden Lösung zur Ausfällung von ABTOX um ca. 30% im Rotationsverdampfer reduziert werden. Es kann in 78,3% (799 mg, 3,91 mmol) Ausbeute isoliert werden. Ähnlich TKX50 wird ABTOX abgenutscht, mit wenig kaltem Wasser gewaschen und an der Luft getrocknet.

TKX50: Dichlorglyoxim (785 mg, 5 mmol) wird in 10 ml N-Methyl-2-pyrrolidon (NMP) bei Raumtemperatur gelöst. Die Lösung wird auf 0°C gekühlt und NaN₃ (715 mg, 11 mmol) zugegeben. Die Mischung wird 40 min bei 0°C gerührt. NaCl fällt aus, wobei Diazidoglyoxim in Lösung bleibt. Die Mischung wird in einen Kolben, in den 150 ml Diethylether vorgelegt wurden, überführt und die Mischung im Salz-Eis-Bad auf 0°C gekühlt. HCl-Gas wird in die Mischung eingeleitet, wobei die Temperatur 20°C nicht überschreiten soll. Eine Sättigung des Ethers mit HCl ist erreicht, sobald die Temperatur trotz fortwährender HCl-Einleitung wieder auf 0-5°C abfällt. Ein dicker Niederschlag, der sich zu Beginn der Gaseinleitung gebildet hat, wird zusehends resuspendiert, wenn HCl-Sättigung erreicht ist. Der Kolben wird fest verschlossen und die Mischung über Nacht bei Raumtemperatur unter leichtem HCl-Überdruck, welcher sich durch das Aufwärmen auf Raumtemperatur im Kolben eingestellt hat, weitergerührt. Der Druck wird abgelassen und die Mischung in ein offenes Gefäß überführt, wo Diethylether und HCl über Nacht bei Raumtemperatur oder in 1-2 h bei 50°C abdampfen können. Nachdem der größte Teil des Diethylethers abgedampft ist, werden 50 ml Wasser zugegeben und das Lösemittel am Rotationsverdampfer wieder entfernt. Der dickflüssige Rückstand, welcher 5,5'-Bistetrazol-1,1'-diol, NaCl und NMP enthält, wird in 20 ml 2M NaOH aufgenommen, wobei das diNatrium-5,5'-bistetrazol-1,1'-diolattetrahydrat auszufallen beginnt. Die Mischung wird kurz aufgekocht und beim Abkühlen fällt das di-Natrium-5,5'-bistetrazol-1,1'-diolattetrahydrat nahezu vollständig aus. Dieses wird abgenutscht und im kleinstmöglichen Volumen kochenden Wassers gelöst (ca. 10 ml). Hydroxylammoniumchlorid (750 mg, 10,8 mmol, 2,16 eq) wird als konzentrierte wässrige Lösung zugegeben. TKX50 fällt aus der Lösung in 85,1% (1,00 g, 4,25 mmol) Ausbeute aus. Dieses wird abgenutscht, mit kaltem Wasser gewaschen und an der Luft getrocknet.

ABTOX: Die Synthesevorschrift orientiert sich an der Synthesevorschrift von TKX50, bis das di-Natrium-5,5'-bistetrazol-1,1'-diolattetrahydrat erhalten wird. Es wird wiederum im kleinstmöglichen Volumen kochenden Wassers gelöst (ca. 10 ml) und Ammoniumchlorid (800 mg, 15,0 mmol, 3eq.) als konzentrierte wässrige Lösung zugegeben. Im Falle von ABTOX muss ein etwas größerer Überschuß Ammoniumsalz zugegeben werden, da die Wasserlöslichkeit von ABTOX etwas größer als die von TKX50 ist. Ebenso muss das Volumen der ABTOX und NaCl enthaltenden Lösung zur Ausfällung von ABTOX um ca. 30% im Rotationsverdampfer reduziert werden. Es kann in 81,3% (830 mg, 4,07 mmol) Ausbeute isoliert werden. Ähnlich TKX50 wird ABTOX abgenutscht, mit wenig kaltem Wasser gewaschen und an der Luft getrocknet.

Die Vorteile der weiteren Syntheseverfahren von TKX50 und ABTOX liegen in erster Linie darin, dass die Isolierung der hoch schlag- und reibeempfindlichen Zwischenstufe Diazidoglyoxim umgangen wird. Die hochsensitive Zwischenstufe bleibt während des gesamten Prozesses in Lösung, so dass keine objektiven Gefahren bei der Synthese entstehen. Die hier isolierten Zwischenstufen Dimethylammonium-5,5'-bistetrazol-1,1'-diolat und di-Natrium-5,5'-bistetrazol-1,1'-diolattetrahydrat zeigen keine wesentliche Schlagempfindlichkeit (beide >40 J) und ebenso keine wesentliche Reibeempfindlickeit (beide >360 N). Ein weiterer Vorteil der hier vorgestellten Synthese ist die Ersparnis zweier Reaktionsschritte, nämlich der Isolierung von Diazidoglyoxim und 5,5'-Bistetrazol-1,1'-dioldihydrat, was einer Darstellung von TKX50 und ABTOX im großindustriellen Maßstab nicht nur aus finanziellen Gründen entgegenkommt.

## Patentansprüche

1. Energetische Wirkmasse umfassend ein Dihydroxylammoniumsalz oder Diammoniumsalz von 5,5'-Bistetrazol-1,1'-diol, 5,5'-Bistetrazol-1,2'-diol oder 5,5'-Bistetrazol-2,2'-diol oder ein Gemisch aus mindestens zwei dieser Salze.

2. Verwendung eines Dihydroxylammoniumsalzes oder Diammoniumsalzes von 5,5'-Bistetrazol-1,1'-diol, 5,5'-Bistetrazol-1,2'-diol oder 5,5'-Bistetrazol-2,2'-diol oder eines Gemischs aus mindestens zwei dieser Salze als Sprengstoff.

3. Verfahren zur Herstellung eines Dihydroxylammoniumsalzes oder Diammoniumsalzes von 5,5'-Bistetrazol-1,1'-diol, 5,5'-Bistetrazol-1,2'-diol oder 5,5'-Bistetrazol-2,2'-diol oder eines Gemischs aus mindestens zwei dieser Salze mit folgenden Schritten:
a) Oxidieren von 5,5'-Bistetrazol zu einem Gemisch aus 5,5'-Bistetrazol-1,1'-diol, 5,5'-Bistetrazol-1,2'-diol und 5,5'-Bistetrazol-2,2'-diol oder
Förderung der Isomerisierung von Diazidoglyoxim zu 5,5'-Bistetrazol-1,1'-diol oder einem 5,5'-Bistetrazol-1,1'-diolat,
b) Inkubation des gemäß Schritt a) erhaltenen Reaktionsprodukts mit Hydroxylamin, Hydroxylammoniumionen, Ammoniumionen oder Ammoniak in wässriger Lösung und
c) Absondern des dabei erhaltenen Präzipitats.

4. Verfahren nach Anspruch 3, wobei das Oxidieren gemäß Schritt a) durch, insbesondere im Überschuss erfolgenden, Zusatz von 2KHSO₅·KHSO₄·K₂SO₄ oder einer anderen anorganischen oder organischen Peroxosäure oder hypofluoriger Säure oder einem anderen Sauerstoffüberträger zum 5,5'-Bistetrazol erfolgt.

5. Verfahren nach Anspruch 3 oder 4, wobei das Oxidieren in einer auf einen pH-Wert zwischen 5 und 8, insbesondere zwischen 7 und 7,5, gepufferten wässrigen Lösung erfolgt.

6. Verfahren nach einem der Ansprüche 3 bis 5, wobei das bei Schritt a) durch Oxidieren von 5,5'-Bistetrazol erhaltene Reaktionsprodukt angesäuert und anschließend mit einem organischen Extraktionsmittel, insbesondere Ether, extrahiert und danach, insbesondere durch Evaporation, aus dem Extrakt gewonnen wird, wobei das Reaktionsprodukt anschließend optional, insbesondere aus Methanol, rekristallisiert wird.

7. Verfahren nach einem der Ansprüche 3 bis 6, wobei bei Schritt a) 5,5'-Bistetrazol oxidiert wird und die wässrige Lösung nach der Präzipitation des Dihydroxylammoniumsalzes oder Diammoniumsalzes von 5,5'-Bistetrazol-1,1'-diol, insbesondere unter Zusatz von weiterem Hydroxylamin oder Ammoniak oder den Hydroxylammonium- oder Ammoniumionen, durch Evaporation eingeengt wird, wobei entweder dadurch zumindest das Löslichkeitsprodukt des Dihydroxylammoniumsalzes oder Diammoniumsalzes von 5,5'-Bistetrazol-1,2'-diol oder 5,5'-Bistetrazol-2,2'-diol überschritten wird, so dass das Dihydroxylammoniumsalz oder Diammoniumsalz präzipitiert oder das Dihydroxylammoniumsalz oder Diammoniumsalz aus dem aus der Evaporation resultierenden Rückstand durch Umkristallisation, insbesondere aus einem Ethanol-Wasser-Gemisch, gewonnen wird.

8. Verfahren nach Anspruch 3, wobei die Förderung der Isomerisierung durch Acylierung, insbesondere mittels Acetylchlorid, oder durch Inkubation mit gasförmigem HCl, insbesondere in Ether als Lösungsmittel oder in einem Ether enthaltenden Lösungsmittel, erfolgt.

9. Verfahren nach Anspruch 3 oder 8, wobei das Diazidoglyoxim durch Reaktion von Dichloroglyoxim mit einem Azid erzeugt wird, wobei das Dichloroglyoxim insbesondere durch Reaktion von Glyoxim mit Chlor erzeugt wird, wobei das Glyoxim insbesondere durch Reaktion von Glyoxal mit Hydroxylamin erzeugt wird.

10. Verfahren nach Anspruch 9, wobei die Reaktion von dem Dichloroglyoxim mit dem Azid in einem nichtwässrigen Lösungsmittel, insbesondere Dimethylformamid (DMF) oder N-Methyl-2-pyrrolidon (NMP), erfolgt, wobei anschließend das Lösungsmittel mit dem dabei entstandenen Diazidoglyoxim mit dem Ether gemischt und mit dem gasförmigen HCl inkubiert wird.

11. Verfahren nach Anspruch 10, wobei vor Schritt c) der Ether und das HCl, insbesondere unter Zusatz von dann ebenfalls abzudampfendem H₂O, abgedampft werden und, sofern vorhanden, das DMF abgedampft wird, so dass im Falle von DMF als Lösungsmittel eine Mischung aus Dimethylammonium-5,5'-bistetrazol-1,1'-diolat und einem 5,5'-Bistetrazol-1,1'-diolat, das mindestens ein Gegenion des Azids umfasst, und im Falle von NMP als Lösungsmittel ein 5,5'-Bistetrazol-1,1'-diol enthaltender Rückstand erhalten wird.

12. Verfahren nach Anspruch 11, wobei das Dimethylammonium-5,5'-bistetrazol-1,1'-diolat in H₂O gelöst wird und anschließend ein Hydroxylammoniumsalz, insbesondere Hydroxylammoniumchlorid, zugesetzt wird, so dass das Dihydroxylammoniumsalz von 5,5'-Bistetrazol-1,1'-diol als das Präzipitat erhalten wird.

13. Verfahren nach Anspruch 11, wobei der 5,5'-Bistetrazol-1,1'-diol enthaltende Rückstand in einer wässrigen Alkalihydroxid-Lösung aufgenommen und das ausfallende Alkali-5,5'-bistetrazol-1,1'-diolat abgesondert und in H₂O gelöst wird, wobei anschließend ein Hydroxylammoniumsalz, insbesondere Hydroxylammoniumchlorid, zugesetzt wird, so dass das Dihydroxylammoniumsalz von 5,5'-Bistetrazol-1,1'-diol als das Präzipitat erhalten wird.

14. Verfahren nach Anspruch 12 oder 13, wobei statt des Hydroxylammoniumsalzes ein Ammoniumsalz, insbesondere Ammoniumchlorid, zugesetzt wird, so dass das Diammoniumsalz von 5,5'-Bistetrazol-1,1'-diol als das Präzipitat erhalten wird.

15. 5,5'-Bistetrazoldiol oder Salz davon, wobei das 5,5'-Bistetrazoldiol ein 5,5'-Bistetrazol-1,2'-diol oder -2,2'-diol ist und das Salz ein Dihydroxylammoniumsalz oder ein Diammoniumsalz ist.

## Claims

1. An energetic active composition comprising a dihydroxylammonium salt or diammonium salt of 5,5'-bistetrazole-1,1'-diol, 5,5'-bistetrazole-1,2'-diol, or 5,5'-bistetrazole-2,2'-diol, or a mixture of at least two of these salts.

2. The use of a dihydroxylammonium salt or diammonium salt of 5,5'-bistetrazole-1,1'-diol, 5,5'-bistetrazole-1,2'-diol, or 5,5'-bistetrazole-2,2'-diol, or a mixture of at least two of these salts, as explosive.

3. A process for preparing a dihydroxylammonium salt or diammonium salt of 5,5'-bistetrazole-1,1'-diol, 5,5'-bistetrazole-1,2'-diol, or 5,5'-bistetrazole-2,2'-diol, or a mixture of at least two of these salts, with the following steps:
a) oxidizing 5,5'-bistetrazole to give a mixture of 5,5'-bistetrazole-1,1'-diol, 5,5'-bistetrazole-1,2'-diol, and 5,5'-bistetrazole-2,2'-diol, or
promoting the isomerization of diazidoglyoxime to 5,5'-bistetrazole-1,1'-diol or a 5,5'-bistetrazole-1,1'-diolate,
b) incubating the reaction product obtained according to step a) with hydroxylamine, hydroxylammonium ions, ammonium ions, or ammonia in aqueous solution, and
c) isolating the resultant precipitate.

4. The process as claimed in claim 3, wherein the oxidizing according to step a) takes place by addition, more particularly addition in excess, of 2KHSO₅·KHSO₄·K₂SO₄ or of another inorganic or organic peroxo acid or hypofluorous acid or another oxygen transfer agent to the 5,5'-bistetrazole.

5. The process as claimed in claim 3 or 4, wherein the oxidizing takes place in an aqueous solution buffered to a pH of between 5 and 8, more particularly between 7 and 7.5.

6. The process as claimed in any of claims 3 to 5, wherein the reaction product obtained in step a) by oxidizing of 5,5'-bistetrazole is acidified and subsequently extracted with an organic extractant, more particularly ether, and thereafter recovered from the extract, more particularly by evaporation, the reaction product being subsequently optionally recrystallized, more particularly from methanol.

7. The process as claimed in any of claims 3 to 6, wherein, in step a), 5,5'-bistetrazole is oxidized and the aqueous solution, after the precipitation of the dihydroxylammonium salt or diammonium salt of 5,5'-bistetrazole-1,1'-diol, more particularly with addition of further hydroxylamine or ammonia or of the hydroxylammonium ions or ammonium ions, is concentrated by evaporation, where either as a result at least the solubility product of the dihydroxylammonium salt or diammonium salt of 5,5'-bistetrazole-1,2'-diol or 5,5'-bistetrazole-2,2'-diol is exceeded, and so the dihydroxylammonium salt or diammonium salt precipitates, or the dihydroxylammonium salt or diammonium salt is obtained from the residue resulting from the evaporation by recrystallization, more particularly from an ethanol/water mixture.

8. The process as claimed in claim 3, wherein the promoting of the isomerization takes place by acylation, more particularly by means of acetyl chloride, or by incubation with gaseous HCl, more particularly in ether as solvent or in an ether-comprising solvent.

9. The process as claimed in claim 3 or 8, wherein the diazidoglyoxime is produced by reaction of dichloroglyoxime with an azide, the dichloroglyoxime being produced more particularly by reaction of glyoxime with chlorine, the glyoxime being produced more particularly by reaction of glyoxal with hydroxylamine.

10. The process as claimed in claim 9, wherein the reaction of the dichloroglyoxime with the azide takes place in a nonaqueous solvent, more particularly dimethylformamide (DMF) or N-methyl-2-pyrrolidone (NMP), the solvent with the resultant diazidoglyoxime being subsequently mixed with the ether and incubated with the gaseous HCl.

11. The process as claimed in claim 10, wherein before step c) the ether and the HCl, more particularly with addition of H₂O which then likewise requires evaporative removal, are evaporated off and, where present, the DMF is evaporated off, and so, in the case of DMF as solvent, a mixture of dimethylammonium 5,5'-bistetrazole-1,1'-diolate and a 5,5'-bistetrazole-1,1'-diolate which comprises at least one counterion of the azide, and in the case of NMP as solvent a residue comprising 5,5'-bistetrazole-1,1'-diol, is obtained.

12. The process as claimed in claim 11, wherein the dimethylammonium 5,5'-bistetrazole-1,1'-diolate is dissolved in H₂O and subsequently a hydroxylammonium salt, more particularly hydroxylammonium chloride, is added, and so the dihydroxylammonium salt of 5,5'-bistetrazole-1,1'-diol is obtained as the precipitate.

13. The process as claimed in claim 11, wherein the residue comprising 5,5'-bistetrazole-1,1'-diol is taken up in an aqueous alkali hydroxide solution and the precipitating alkali 5,5'-bistetrazole-1,1'-diolate is isolated and dissolved in H₂O, with subsequent addition of a hydroxylammonium salt, more particularly hydroxylammonium chloride, and so the dihydroxylammonium salt of 5,5'-bistetrazole-1,1'-diol is obtained as the precipitate.

14. The process as claimed in claim 12 or 13, wherein an ammonium salt, more particularly ammonium chloride, is added instead of the hydroxylammonium salt, and so the diammonium salt of 5,5'-bistetrazole-1,1'-diol is obtained as the precipitate.

15. 5,5'-Bistetrazolediol or a salt thereof, wherein the 5,5'-bistetrazolediol is a 5,5'-bistetrazol-1,2'-diol or a -2,2'-diol and the salt is a dihydroxylammonium salt or a diammonium salt.

## Revendications

1. Masse active énergétique comprenant un sel de dihydroxylammonium ou sel de diammonium de 5,5'-bistétrazole-1,1'-diol, 5,5'-bistétrazole-1,2'-diol ou 5,5'-bistétrazole-2,2'-diol ou un mélange d'au moins deux de ces sels.

2. Utilisation d'un sel de dihydroxylammonium ou sel de diammonium de 5,5'-bistétrazole-1,1'-diol, 5,5'-bistétrazole-1,2'-diol ou 5,5'-bistétrazole-2,2'-diol ou d'un mélange d'au moins deux de ces sels comme explosif.

3. Procédé de fabrication d'un sel de dihydroxylammonium ou sel de diammonium de 5,5'-bistétrazole-1,1'-diol, 5,5'-bistétrazole-1,2'-diol ou 5,5'-bistétrazole-2,2'-diol ou d'un mélange d'au moins deux de ces sels avec les étapes suivantes:
a) oxydation de 5,5'-bistétrazole en un mélange de 5,5'-bistétrazole-1,1'-diol, 5,5'-bistétrazole-1,2'-diol et 5,5'-bistétrazole-2,2'-diol ou
promotion de l'isomérisation de diazidoglyoxime en 5,5'-bistétrazole-1,1'-diol ou un 5,5'-bistétrazole-1,1'-diolate,
b) incubation du produit de réaction obtenu selon l'étape a) avec de l'hydroxylamine, des ions hydroxylammonium, des ions ammonium ou de l'ammoniaque en solution aqueuse et
c) séparation du précipité obtenu ce faisant.

4. Procédé selon la revendication 3, dans lequel l'oxydation selon l'étape a) s'effectue par ajout, s'effectuant notamment en excès, de 2KHSO₅·KHSO₄·K₂SO₄ ou d'un autre peroxoacide inorganique ou organique ou acide hypofluoré ou un autre vecteur d'oxygène au 5,5'-bistétrazole.

5. Procédé selon la revendication 3 ou 4, dans lequel l'oxydation s'effectue dans une solution aqueuse tamponnée à une valeur de pH entre 5 et 8, notamment entre 7 et 7,5.

6. Procédé selon une des revendications 3 à 5, dans lequel le produit de réaction obtenu à l'étape a) par oxydation de 5,5'-bistétrazole est acidifié, puis extrait avec un agent d'extraction organique, notamment de l'éther, et est ensuite obtenu à partir de l'extrait, notamment par évaporation, dans lequel le produit de réaction est ensuite recristallisé en option, notamment à partir de méthanol.

7. Procédé selon une des revendications 3 à 6, dans lequel du 5,5'-bistétrazole est oxydé à l'étape a) et la solution aqueuse est concentrée après la précipitation du sel de dihydroxylammonium ou sel de diammonium de 5,5'-bistétrazole-1,1'-diol, notamment en ajoutant de l'hydroxylamine ou de l'ammoniaque supplémentaire ou des ions hydroxylammonium ou ammonium, par évaporation, dans lequel soit au moins le produit de solubilité du sel de dihydroxylammonium ou sel de diammonium de 5,5'-bistétrazole-1,2'-diol ou 5,5'-bistétrazole-2,2'-diol est dépassé de ce fait de sorte que le sel de dihydroxylammonium ou sel de diammonium est précipité, soit le sel de dihydroxylammonium ou sel de diammonium est obtenu à partir du résidu résultant de l'évaporation par recristallisation, notamment à partir d'un mélange d'éthanol-eau.

8. Procédé selon la revendication 3, dans lequel la promotion de l'isomérisation s'effectue par acylation, notamment au moyen de chlorure d'acétyle, ou par incubation avec du HCl gazeux, notamment dans de l'éther comme solvant ou dans un solvant contenant de l'éther.

9. Procédé selon la revendication 3 ou 8, dans lequel le diazidoglyoxime est généré par réaction de dichloroglyoxime avec un azoture, dans lequel le dichloroglyoxime est notamment généré par réaction de glyoxime avec du chlore, dans lequel le glyoxime est notamment généré par réaction de glyoxal avec de l'hydroxylamine.

10. Procédé selon la revendication 9, dans lequel la réaction du dichloroglyoxime avec l'azoture s'effectue dans un solvant non aqueux, notamment du diméthylformamide (DMF) ou de la N-méthyl-2-pyrrolidone (NMP), dans lequel le solvant est ensuite mélangé avec le diazidoglyoxime apparu ce faisant avec l'éther et incubé avec le HCl gazeux.

11. Procédé selon la revendication 10, dans lequel l'éther et le HCl sont évaporés avant l'étape c), notamment en ajoutant du H₂O à évaporer également alors, et, dans la mesure où il est présent, le DMF est évaporé de sorte que, dans le cas de DMF comme solvant, un mélange de diméthylammonium-5,5'-bistétrazole-1,1'-diolate et d'un 5,5'-bistétrazole-1,1'-diolate qui comprend au moins un ion conjugué de l'azoture, et, dans le cas de NMP comme solvant, un résidu contenant du 5,5'-bistétrazole-1,1'-diol est obtenu.

12. Procédé selon la revendication 11, dans lequel le diméthylammonium-5,5'-bistétrazole-1,1'-diolate est dissous dans du H₂O, puis un sel d'hydroxylammonium, notamment chlorure d'hydroxylammonium, est ajouté de sorte que le sel de dihydroxylammonium de 5,5'-bistétrazole-1,1'-diol est obtenu comme le précipité.

13. Procédé selon la revendication 11, dans lequel le résidu contenant du 5,5'-bistétrazole-1,1'-diol est absorbé dans une solution d'hydroxyde alcalin aqueuse, et le 5,5'-bistétrazole-1,1'-diolate alcalin précipité est séparé et dissous dans du H₂O, dans lequel un sel d'hydroxylammonium, notamment chlorure d'hydroxylammonium, est ensuite ajouté de sorte que le sel de dihydroxylammonium de 5,5'-bistétrazole-1,1'-diol est obtenu comme le précipité.

14. Procédé selon la revendication 12 ou 13, dans lequel un sel d'ammonium, notamment chlorure d'ammonium, est ajouté, au lieu du sel d'hydroxylammonium, de sorte que le sel de diammonium de 5,5'-bistétrazole-1,1'-diol est obtenu comme le précipité.

15. 5,5'-Bistétrazolediol ou sel de celui-ci, dans lequel le 5,5'-bistétrazolediol est un 5,5'-bistétrazole-1,2'-diol ou -2,2'-diol et le sel est un sel de dihydroxylammonium ou un sel de diammonium.
